Europäisches Patentamt

European Patent Office

Office européen des brevets

(11) Publication number: **0 132 105**
**B1**

# EUROPEAN PATENT SPECIFICATION

(45) Date of publication of patent specification: **31.10.90**

(51) Int. Cl.⁵: **A 61 K 31/71,** A 61 K 47/00

(21) Application number: **84304702.8**

(22) Date of filing: **10.07.84**

(54) Oily composition of aclarubicin or hydrochloride thereof.

(30) Priority: **13.07.83 JP 127092/83**

(43) Date of publication of application:
**23.01.85 Bulletin 85/04**

(45) Publication of the grant of the patent:
**31.10.90 Bulletin 90/44**

(84) Designated Contracting States:
**DE FR GB IT**

(56) References cited:
EP-A-0 058 387
CH-A- 229 981
FR-A-2 498 075
FR-A-2 519 864

(73) Proprietor: YAMANOUCHI PHARMACEUTICAL
CO. LTD.
No. 3-11 Nihonbashi-Honcho, 2-chome Chuo-ku
Tokyo (JP)

(72) Inventor: Kawata, Hiroitsu
No.1-7, Oaza Namiki
Kawagoe-shi Saitama (JP)
Inventor: Hasumi, Shunji
No.4-284, Futatsuya
Kitamoto-shi Saitama (JP)
Inventor: Okada, Akira
No.1-16-6, Fujimi
Ageo-shi Saitama (JP)
Inventor: Aruga, Masayoshi
No.5-16-14, Modorigaoka
Ageo-shi Saitama (JP)
Inventor: Konno, Toshimitsu
No.6-581, Izumi
Kumamoto-shi Kumamoto (JP)
Inventor: Iwai, Ken
No.1105-41, Taniozaki-cho
Kumamoto-shi Kumamoto (JP)
Inventor: Maeda, Hiroshi
No.631-3, Aza-Tamukae Hotakubohonmachi
Kumamoto-shi Kumamoto (JP)

Courier Press, Leamington Spa, England.

**EP 0 132 105 B1**

(74) Representative: **Geering, Keith Edwin et al**
**REDDIE & GROSE 16 Theobalds Road**
**London WC1X 8PL (GB)**

# EP 0 132 105 B1

**Description**

This invention relates to an oily composition of aclarubicin or the hydrochloride thereof.

If an antitumor drug for treating neoplastic tissues is orally or intravenously administered, it diffuses into the whole body and its concentration in the neoplastic tissues is low; hence sufficient treatment effect cannot be obtained and undesirable side effects on the living body sometimes occur.

For overcoming these problems, it has been proposed to administer a preparation of an antitumor drug on a carrier having low toxicity such as albumin, synthetic high mole. wt. materials, fats and oils, directly into a lymph vessel or blood vessel for the internal organ to be treated.

However, since many important antitumor drugs are water-soluble or sparingly soluble in oils, it is generally difficult to produce preparations of antitumor drugs carried on fats and oils for the above-described administration method. In fact, the only such preparation used in practice is a preparation of epitiostanol dissolved in sesame oil.

We previously developed oily preparations of sparingly oil soluble or water-soluble antitumor drugs for the above-described administration method, using solubilising adjuvants such as crown ethers (see U.K. 2 114 885 A).

We have now found that the specific antitumor drug, aclarubicin or aclarubicin hydochloride, can be dissolved well in particular fatty acids and fats and oils without using solubilising adjuvants such as crown ether.

According to this invention, there is provided an oil composition which is a solution comprising aclarubicin and/or aclarubicin hydrochloride in at least one fatty acid selected from saturated medium chain ($C_6$ to $C_{10}$, preferably $C_8$ to $C_{10}$) fatty acids and medium and long chain ($C_6$ and longer) unsaturated fatty acids, and at least one fat or oil selected from iodized oils and vegetable oils. The chain lengths quoted above include the carboxy carbon.

Aclarubicin is a known antitumor antibiotic of the anthracycline series, obtainable by the cultivation of Streptomyces galilaeus and by other methods, and is expressed by the chemical name methyl(1R,2R,4S) - [2,6 - dideoxy - 4 - O - ((2R,6S - tetrahydro - 6 - methyl - 5 - oxo - 2H - pyran - 2 - yl) - α - 1 - lyxo - hexopyranocyl] - 3 - dimethylamino - α - 1 - lyxo - hexopyranocyl] - oxy} - 1 - naphthacene carboxylate. Usually, the antibiotic is used as aclarubicin hydrochloride. Usually, the antibiotic is used as aclarubicin hydrochloride.

Aclarubicin hydrochloride is freely soluble in water, acetone, methanol, etc., but is practically insoluble in ether and n-hexane. Aclarubicin hydrochloride is very sparingly soluble in fats and oils. For example, the solubility of aclarubicin hydrochloride in sesame oil is 16 µg/ml and that in olive oils is about 4 µg/ml.

Aclarubicin is sparingly soluble in sesame oil (10 mg/ml) and in olive oil (10 mg/ml), but the composition of this invention can be dissolved in these oils to an aclarubicin solubility about 5 times higher than this. Accordingly, an antitumor drug solution having a high concentration can be prepared by using the composition of this invention and hence the antitumor drug can reach the special neoplastic tissues at high concentration to increase the treatment effect.

In this invention, saturated or unsaturated medium chain fatty acids or unsaturated long chain fatty acids can be used as fatty acids. The medium chain fatty acids include capric acid, caproic acid, caprylic acid, caproleinic acid, etc., and unsaturated long chain fatty acids include linoleic acid, oleic acid, linolenic acid, etc. These fatty acids may be used singly or in admixture.

Lower fatty acids may solubilize aclarubicin or aclarubicin hydrochloride in fats and oils, but since lower fatty acids have high solubility in water and a large dissociation constant, they cannot be used as they are. On the other hand, saturated long chain fatty acids (e.g., palmitic acid and stearic acid) have a very low solubilizing faculty for aclarubicin or aclarubicin hydrochloride in fats and oils and are solid. Therefore, when saturated long chain fatty acids are used in large quantities, the system is solidified and cannot be used for practical purposes. It has also found that polycarboxylic acids such as fumaric acid, tartaric acid, succinic acid, etc., do no solubilise aclarubicin or aclarubicin hydrochloride in fats and oils.

Only when the fatty acids specified for this invention are used can sufficient solubilisation of aclarubicin or aclarubicin hydrochloride in fats and oils be achieved and the desired oily compositions of these antitumor agents be obtained.

As fats and oils in this invention, iodized oils or vegetable oils are used. Iodized oils include iodized poppy seed oil fatty acid ethyl ester (e.g. Lipiodol Ultra-Fluide, trade name, made by Laboratoire Guerbert), strong iodized oil (the 9th revised Japanese Pharmacopoeia), iodized oil (the 9th revised Japanese Pharmacopoeia), iodized medium chain or long chain fatty acids, etc.; and vegetable oils include sesame oil, peanut oil, cotton seed oil, olive oil, almond oil, etc.

The reason for using these fats and oils in this invention is that when administered in the artery serving the tumor portion, the fats and oils retained at the tumor portion; when, in particular, iodized oil is used, diagnosis of the tumor portion becomes possible [Toshimitsu Konno et al "Gan to Kagaku Ryoho (Cancer and Chemotherapy"), 9 (11), 2005—2015 (1982) and Kenichiro Nakakuma et al, "Nichidoku Iho (Japan and Germany Medical Report):, 24(4), 675—682 (1979)]. Among these fats and oils, iodized poppy seed oil fatty acid ethyl ester is particularly preferred.

The compounding ratio of fatty acid in the composition of this invention is e.g. 10—1,000 parts by weight (preferably 30—200 parts by weight) per 1 part by weight of aclarubicin hydrochloride and 1—1,000 parts by weight (preferably 1—50 parts by weight) per 1 part by weight of aclarubicin.

The composition of this invention may further contain an antioxidant (α-tocophenol, etc.,) and other additives.

The composition of this invention can be prepared by mixing aclarubicin or aclarubicin hydrochloride with at least one of the above-described fatty acids and at least one of the above-described fats and oils. Shaking or stirring may be needed to ensure dissolution. In particular, when a vegetable oil is used, dissolution may be performed using a stirrer mixer or ultrasonic wave bath, etc.

Alternatively the components may be converted into a solution only at the time of use. Thus aclarubicin hydrochloride may be dissolved in water (or aclarubicin in an appropriate organic solvent such as 1,4-dioxane) and the solution is lyophilized in an appropriate vessel (e.g. a vial); at least one of the above-described fatty acids and at least one of the above-described fats and oils are mixed and the liquid is placed in an appropriate container such as an ampoule. For use, the liquid is added to the lyophilized antitumor and the antitumor is dissolved to provide a liquid composition of this invention.

A suitable concentration of aclarubicin hydrochloride and/or aclarubicin in the composition of this invention is 1 mg (potency)/1 ml to 100 mg (potency)/1 ml.

The antitumor effect of compositions according to the invention on New Zealand white rabbits is shown by the following experiment.

Experimental procedure:

The abdominal cavity of each rabbit was opened under anesthesia with Pentobarbital and 0.1 ml of a suspension containing 1,000,000 to 1,500,000 VX$_2$ cancer cells in 1 ml of the suspension was injected into the suberosal tissue of the left lateral lobe of the liver. Two weeks after the injection, it was confirmed tbat the tumor size became 1 to 2 cm and then respective groups of the rabbits were treated as follows, the injections in each case being into the common hepatic artery under laparotomy.

Group I: Aclarubicin hydrochloride oily solution-administered group (4 rabbits).

0.2 ml of the aclarubicin hydrochloride oily solution (12.5 mg/ml) obtained in Example 12 below was injected into each rabbit.

The following groups were injected in the same manner as for Group I.

Group II: Aclarubicin oily solution-administered group (4 rabbits).

0.2 ml of the aclarubicin oily solution (25 mg/ml) obtained in Example 13 below was injected into each rabbit.

Group III: Group simultaneously administered with aqueous aclarubicin hydrochloride solution and Lipiodol Ultra-Fluide (4 rabbits).

0.25 ml of aqueous aclarubicin hydrochloride solution (20 mg/ml) was injected into each rabbit and immediately thereafter 0.2 ml of Lipiodol Ultra-Fluide was injected into each rabbit.

Group IV: Aqueous aclarubicin hydrochloride solution-administered group (3 rabbits):

2.5 ml of an aqueous aclarubicin hydrochloride solution (20 mg/ml) was injected into each rabbit.

Group V: Lipiodol Ultra-Fluide slone-administered group (4 rabbits).

0.2 ml of Lipiodol Ultra-Fluide was injected into each rabbit.

A week after the injections, the rabbits were sacrificed and Softex radiographs and histological analyses were performed.

Experimental results:

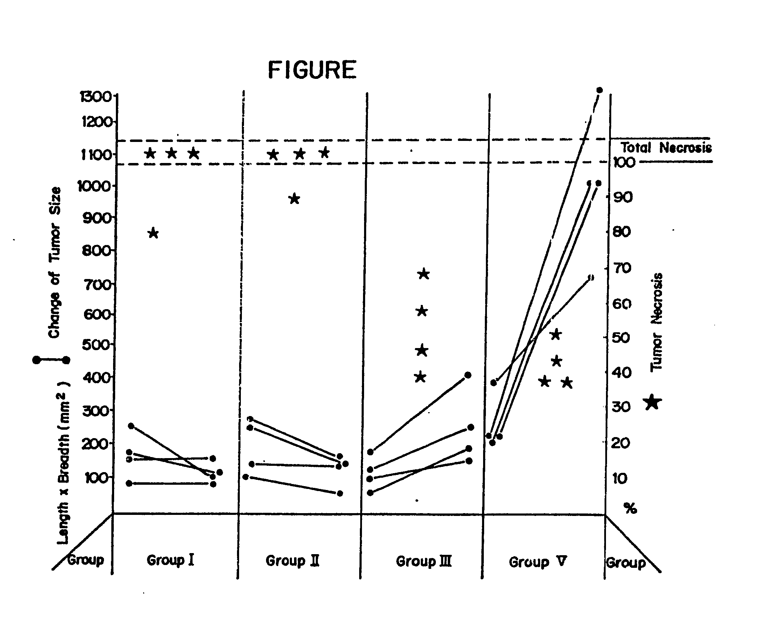

The change (—●) in tumor size and the %age ( ★ ) tumor necrosis of the rabbits from group I to group V are shown in the accompanying Figure.

Using Lipiodol Ultra-Fluide alone, a little the necrosis caused by the embolization of new blood vessels was confirmed but total necrosis was not obtained and the tumor size increased. On the other hand, in the aclarubicin hydrochloride oily solution-administered group and the aclarubicin oily solution-administered group, increase of tumor size was not observed, and total necrosis was observed in ¾ of the rabbits — that is, the tumor portions changed into light grey and were softened, which showed that the tumor portions were mostly in the degenerative and necrotic states in pathalogical tissues. At the periphery of the tumor, remarkable inflammatory cell infiltration, in particular lymphocytic infiltration was observed.

In Group III, simultaneously administered with an aqueous aclarubicin hydrochloride solution and Lipiodol Ultra-Fluide, a little necrosis was observed, but the result was almost the same as when using Lipiodol Ultra-Fluide alone and viable cancer cells were observed in both cases. From the X-ray photographs, in Group I, Group II, Group III and Group V, Lipiodol Ultra-Fluide was observed only at the tumor portions in each case. Also, in Group IV wherein an aqueous aclarubicin hydrochloride solution was administered in an amount 20 times (as aclarubicin) that of the oily solution of aclarubicin, a necrosis phase was oberved at the center of the tumor portion but viable cancer cells were observed in each case.

From the above experimental results, it is clear that the composition of this invention wherein aclarubicin hydrochloride or aclarubicin is solubilized in Lipiodol Ultra-Fluide shows improved antitumor effect as compared with the case of administering Lipiodol Ultra-Fluide alone, the case of administering an aqueous aclarubicin hydrochloride solution of 20 times (as aclarubicin hydrochloride) the oily solution strength, and the case of simultaneously administering an aqueous aclarubicin hydrochloride solution and Lipiodol Ultra-Fluide.

The invention is further illustrated by the following examples.

4

## Example 1

To 1 g (potency) of aclarubicin hydrochloride were added 22.6 g of linoleic acid and 96.2 g of an iodized poppy seed oil fatty acid ethylester (Lipiodol Ultra-Fluide, trade name) and the mixture was shaken to provide a transparent solution. The iodized component in subsequent Examples is the same (Lipiodol Ultra-Fluide) as in Example 1.

## Example 2

To 20 mg (potency) of aclarubicin hydrochloride were added 0.5 g of oleic acid and 1.5 ml of the iodized poppy seed oil fatty acid ethylester (same as in Example 1) and the mixture was shaken to provide a transparent solution.

## Example 3

To 30 mg (potency) of aclarubicin hydrochloride were added 500 g of n-capric acid and 1.5 ml of the iodized poppy seed oil fatty acid ethylester and the mixture was shaken to provide a transparent solution.

## Example 4

To 100 mg (potency) of aclarubicin hydrochloride were added 1.8 g of linoleic acid, 200 ml of the egg lecithin, 50 mg of α-tocopherol, and 7.9 g of the iodized poppy seed oil fatty acid ethylester (as in Example 1) and the mixture was shaken to provide a transparent solution.

## Example 5

To 50 mg (potency) of aclarubicin were added 0.5 ml of linoleic acid and 1.5 ml of the iodized poppy seed oil fatty acid ethylester (as in Example 1) and the mixture was shaken to provide a transparent solution.

## Example 6

To 50 mg (potency) of aclarubicin were added 100 mg of n-caproic acid and 1 ml of the iodized poppy seed oil fatty acid ethylester (as in Example 1) and the mixture was shaken to provide a transparent solution.

## Example 7

In 1,4-dioxane was dissolved 50 mg (potency) of aclarubicin and the solution was lyophilized. To the lyophilized product was added a mixture of 0.18 g of linoelic acid and 2.31 g of the iodized poppy seed oil fatty acid ethylester (as in Example 1), whereby aclarubicin was quickly dissolved to provide a transparent solution.

## Example 8

To 50 mg (potency) of aclarubicin were added 50 mg of α-tocopherol and further 0.5 ml of oleic acid and 1.5 ml of the iodized poppy seed oil fatty acid ethylester (as in Example 1), and the mixture was shaken to provide a transparent solution.

## Example 9

To 53 mg (potency) of aclarubicin were added 0.5 ml of linolenic acid and 1 ml of sesame oil and the mixture was immersed in a ultra sonicator bath at 80 watts for 10 minutes to provide a transparent solution.

## Example 10

To 50 mg (potency) of aclarubicin were added 0.2 ml of oleic acid and further 1 ml of peanut oil and the mixture was shaken to provide a transparent solution.

## Example 11

To 25 mg (potency) of aclarubicin hydrochloride were added 0.5 ml of linoleic acid and 0.2 ml of sesame oil and the mixture was immersed in a ultra sonicator bath at 80 watts for 10 minutes to provide a transparent solution.

## Example 12

To 2 g (potency) of aclarubicin hydrochloride were added 72 g of linoleic acid and 103 g of the iodized poppy seed oil fatty acid ethylester and the mixture was shaken vigorously to provide a transparent solution.

## Example 13

To 3.0 g (potency) of aclarubicin were added 22 g of linoleic acid and 120 g of the iodized poppy seed oil fatty acid ethylester and the mixture was shaken vigorously by means of a shaker to provide a transparent solution.

The solutions obtained in the above-described examples are used as injections.

**Claims**

1. An oily antitumor composition which is a solution comprising aclarubicin and/or aclarubicin hydrochloride in at least one fatty acid selected from capric, caproic, caprylic, caproleinic, oleic, linoleic and linolenic acids and at least one fat or oil selected from iodized oils and vegetable oils.

2. An oily antitumor composition which is a solution comprising aclarubicin and/or aclarubicin hydrochloride in at least one fatty acid selected from saturated $C_6$ to $C_{10}$ fatty acids and unsaturated medium and long chain fatty acids of six or more carbon atoms and at least one fat or oil selected from iodized oils and vegetable oils.

3. A solution according to claim 2 which contains no solubiliser selected from crown ethers, lecithin, polyethylene glycol, propylene glycol, vitamin E, alcoholic ethers of polyoxyethylene and sucrose esters of fatty acids.

4. A solution according to claim 1 or 2 wherein the compounding ratio of the fatty acid is 10 to 1,000 parts by weight per 1 part by weight of aclarubicin hydrochloride or 1 to 1,000 parts by weight per 1 part by weight of aclarubicin.

5. A solution according to any preceding claim wherein the total concentration of aclarubicin and/or aclarubicin hydrochloride is 1 mg (potency) to 100 mg (potency) per 1 ml of the composition.

**Patentansprüche**

1. Öliges Antitumormittel, das eine Lösung ist, umfassend Aclarubicin und/oder Aclarubicinhydrochlorid in mindestens einer Fettsäure ausgewählt aus Caprin-, Capron-, Capryl-, Caprolein-, Öl, Linol- und Linolensäure, und mindestens ein Fett oder Öl ausgewählt aus iodierten Ölen und pflanzlichen Ölen.

2. Öliges Antitumormittel, das eine Lösung ist, umfassend Aclarubicin und/oder Aclarubicinhydrochlorid in mindestens einer Fettsäure ausgewählt aus gesättigten $C_6$- bis $C_{10}$-Fettsäuren und ungesättigten mittel- und langkettigen Fettsäuren mit sechs oder mehr Kohlenstoffatomen, und mindestens ein Fett oder Öl ausgewählt aus iodierten Ölen und pflanzlichen Ölen.

3. Lösung nach Anspruch 2, die keinen Löslichmacher, ausgewählt aus Kronenethern, Lecithin, Polyethylenglykol, Popylenglykol, Vitamin E, Alkoholethern von Polyoxyethylen und Saccharoseester von Fettsäuren enthält.

4. Lösung nach Anspruch 1 oder 2, wobei das Mischungsverhältnis der Fettsäure 10 bis 1000 Gew.-Teile auf 1 Gew.-Teil Aclarubicinhydrochlorid oder 1 bis 1000 Gew.-Teile auf 1 Gew.-Teil Aclarubicin beträgt.

5. Lösung nach einem der vorangehenden Ansprüche, wobei die Gesamtkonzentration eine Aclarubicin und/oder Aclarubicinhydrochlorid 1 mg (Wirksamkeit = potency) bis 100 mg (Wirksamkeit = potency) pro 1 ml des Mittels beträgt.

**Revendications**

1. Composition antitumorale huileuse qui est une solution comprenant de l'aclarubicine et/ou du chlorhydrate d'aclarubicine dans au moins un acide gras choisi parmi les acides caprique, caproïque, caprylique, caproléinique, oléique, linoléique et linolénique et au moins une graisse ou huile choisie parmi les huiles iodées et les huiles végétales.

2. Composition antitumorale huileuse qui est une solution comprenant de l'aclarubicine et/ou du chlorhydrate d'aclarubicine dans au moins un acide gras choisie parmi les acides gras saturés en $C_6$ à $C_{10}$ et les acides gras insaturés a chaîne moyenne et à longue chaîne, ayant six atomes de carbone ou avantage, et au moins une graisse ou huile choisie parmi les huiles iodées et les huiles végétales.

3. Solution selon la revendication 2, qui ne contient pas de solubilisant choisi parmi les éthers couronnes, la lécithine, le polyéthylene glycol, le propylène glycol, la vitamine E, les éthers alcooliques de polyoxyéthylène et les esters de sucrose et d'acides gras.

4. Solution selon l'une des revendications 1 ou 2 ou 3, dans laquelle le rapport de formulation de l'acide gras est de 10 à 1000 parties en poids pour 1 partie en poids de chlorhydrate d'aclarubicine ou 1 à 1000 parties en poids pour 1 partie en poids d'aclarubicine.

5. Solution selon l'une des revendications précédentes, dans laquelle la concentration totale d'aclarubicine et/ou chlorhydrate d'aclarubicine est de 1 mg (puissance) à 100 mg (puissance) pour 1 ml de la composition.

FIGURE